# EUROPEAN PATENT APPLICATION

(11) **EP 1 736 097 A1**
(43) Date of publication of application: **27.12.2006**
(21) Application number: 06076089.9
(22) Date of filing: 23.05.2006
(51) Int. Cl.: A61B 3/12, A61B 3/15

(54) **Ophthalmic apparatus**

(30) Priority: 26.05.2005 JP 2005153782
(71) Applicant: Kabushiki Kaisha TOPCON, Tokyo 174-8580 (JP)
(72) Inventor: Kogawa, Taisaku, c/o Kabushiki Kaisha Topcon, Tokyo 174-8580 (JP); Sugino, Yuichi, c/o Kabushiki Kaisha Topcon, Tokyo 174-8580 (JP)
(74) Representative: Pfenning, Meinig & Partner GbR

(57) **Abstract**

An ophthslmic apparatus including a photographic optical system (31, 48) capable of photographing a tested eye (E) and obtaining an image of the tested eye, a drive device (76, 83, 90) configured to drive the photographic optical system relative to the tested eye in three-dimensions, an alignment target projecting optical system (56) to project alignment light flux to the tested eye, and a control device (84) configured to control the drive device based on a state of reflection light flux formed by reflection of the alignment light flux projected by the alignment target projecting optical system on the tested eye, when a flare occurs in the reflection light flux of the alignment light flux reflected on the tested eye, the control device being configured to control the drive device to move the photographic optical system in a direction where the flare decreases.

## Description

### BACKGROUND OF THE INVENTION

### Field of the Invention

The present invention relates to an ophthalmic apparatus which is configured to project alignment light flux to a tested eye and adjust a position of a photographic optical system photographing the tested eye relative to the tested eye based on a state of reflection light flux of the alignment light flux reflected on the tested eye.

### Description of Related Art

Conventionally known is a fundus camera or the like which is configured to project alignment light flux on a portion of cornea of a tested eye, form an alignment image or an image of bright point by reflection light flux of the alignment light flux reflected on the cornea, and execute alignment between the tested eye and a photographical optical system to photograph the tested eye (see, for reference, Japanese Patent Laid-Open 11-4808).

In such a fundus camera, after the alignment of the photographical optical system relative to the tested eye is completed, the photograph of fundus is executed by imaging the photographic optical system on the fundus of the tested eye.

However, even if a subject faces a body of the fundus camera, the tested eye tends to face the photographic optical system substantially orthogonally because the tested eye has heterophoria or the like. In this case, even if a working distance or operational distance from tested eye to the fundus camera body is adequate, an optical axis of the photographic optical system is deviated from an optical axis of the tested eye, hence the alignment light flux enters the cornea of the tested eye obliquely.

As a result, there is a problem that flare occurs in the reflection light flux to affect a fundus image.

Moreover, if the cornea of the tested eye transforms because of cornea disease or the like, when the alignment light flux is reflected on the cornea, flare occurs. The flare experts a negative impact on the fundus image.

Furthermore, if pupil of the tested eye is lesser than the standard, even if a central portion of the fundus is photographed, the alignment light flux is interrupted by iris to generate flare in a circumference portion of the fundus image. If such flare occurs in the fundus image, there is a problem that a diseases part of the fundus cannot be diagnosed.

### SUMMRY OF THE INVENTION

An object of the present invention is to provide an ophthalmic apparatus capable of performing alignment of a photographic optical system without exerting a negative impact on the diagnosing of a disoasc part of fundus even if flare occurs in a fundus image.

To accomplish the above object, an ophthalmic apparatus according to one embodiment of the present invention includes a photographic optical system capable of photographing a tested eye and obtaining an image of the tested eye, a drive device configured to drive the photographic optical system relative to the tested eye in three-dimensions, an alignment target projecting optical system to project alignment light flux to the tested eye, and a control device configured to control the drive device based on a state of reflection light flux formed by reflection of the alignment light flux projected by the alignment target projecting optical system on the tested eye.

The control device is configured, when flare occurs in the reflection light flux of the alignment light flux reflected on the tested eye, to control the drive device to move the photographic optical system in a direction where the flare decreases.

### BRIEF DESCRIPTION OF THE DRAWINGS

FIG.1A is a perspective view showing an ophthalmic apparatus according to the present invention with viewed from a left side.
FIG.1B is a perspective view showing the ophthalmic apparatus according to the present invention with viewed from a right side.
FIG.2 is a systematic diagram showing one embodiment of a photographic optical system and so on of the ophthalmic apparatus according to the present invention.
FIG.3 is a schematic view showing a state connecting a control device in the ophthalmic apparatus as shown in FIGs.1A and 1B and other parts.
FIG.4 is a plan view of a two opening-aperture stop shown in FIG.2.
FIG.5 is a graph showing a translucent characteristic of a half mirror shown in FIG.2.
FIG.6A is a plan view showing a fundus image observed by use of the ophthalmic apparatus when an operational distance between a tested eye and a main body is adequate.
FIG.6B is a plan view showing the fundus image observed by use of the ophthalmic apparatus when the operational distance between the tested eye and the main body is not adequate.

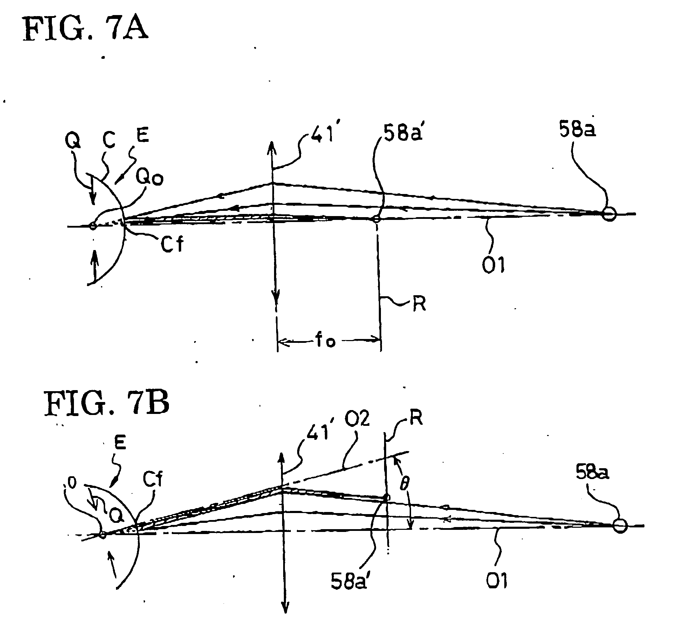

FIG.7A is an explanatory view showing a state in which an alignment image is formed on a fundus conjugate plane on an optical axis of the photographic optical system when an optical axis of the tested eye is aligned with the optical axis of the photographic optical system.
FIG.7B is an explanatory view showing a state in which the alignment image is formed on the fundus conjugate plane out of the optical axis of the photographic optical system when the optical axis of the photographic optical system is inclined at a predetermined angle relate to the optical axis of the tested eye.
FIG.8 is an explanatory view showing a relation between defection of the fundus image and flare.
FIG.9 is an explanatory view of an operating switch designating a generated position of flare of the fundus image as shown in FIG.8.
FIG.10 is an explanatory view showing a panorama fundus image.
FIG.11 is an explanatory view showing the designation of generated flare position in the case of forming the panorama fundus image as shown in FIG. 10.
FIG.12 is an explanatory view showing a modified ophthalmic apparatus according to the present invention.
FIG.13 is a schematic view showing a state connecting a control device in the ophthalmic apparatus as shown in FIG.12 and other parts.
FIG.14 is an explanatory view of a panorama fundus image showing other condition for flare generation in the ophthalmic apparatus according to the present invention.

### DETAILED DESCRIPTION OF THE PREFERRED EMBODIMENTS

Preferred embodiments of the present invention will be explained in detail with reference to the accompanying drawings below.

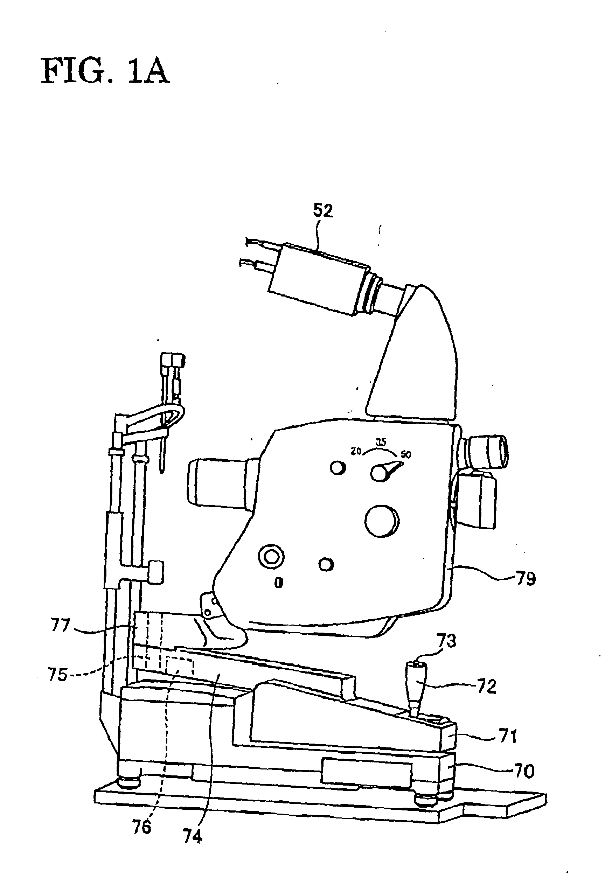

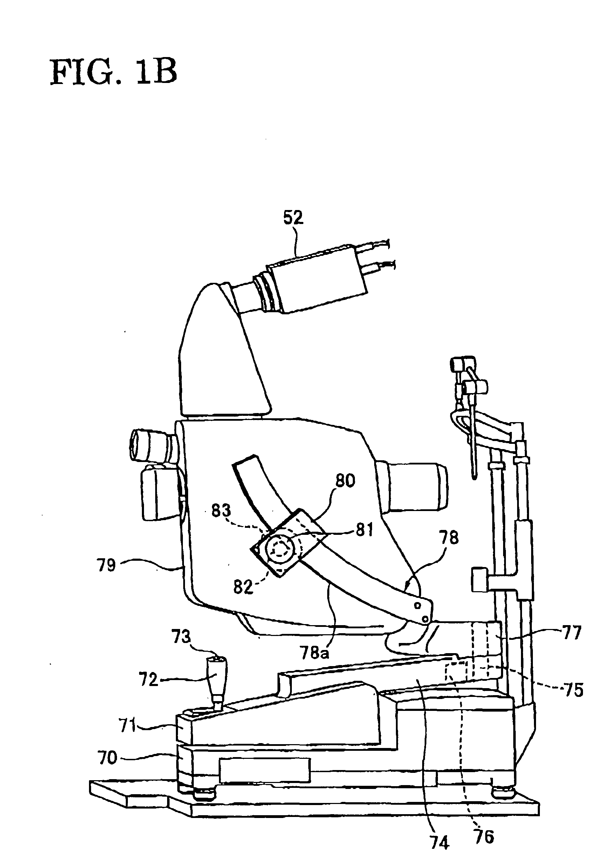

FIGs.1A and 1B illustrate an ophthalmic apparatus according to the present invention. The ophthalmic apparatus includes a fixed base 70, a movable base 71 mounted on the fixed base 70 to be capable of moving from front to back and from side to side, and a joystick 72 shiftably attached to the movable base 71 for operating the movable base movably back and forth and around. The joystick 72 has a top portion on which a photographing switch 3 is provided.

The movable base 71 includes a backwardly and forwardly extending arm 74. The arm 74 is provided movably upward and downward by a pulse motor or the like (not shown) when operating the joystick 72.

The ophthalmic apparatus also includes a supporting shaft 75 rotatably attached to a front end portion of the arm 74, a horizontally rotary motor 76 to rotate the supporting shaft 75 horizontally through a gear mechanism (not shown), and a horizontally rotated member 77 fixed to an upper portion of the supporting shaft 75.

A guide arm 78 which extends in a circular-arc shape as shown in FIG.1B and has a convex surface 78a disposed downwardly is fixed to the horizontally rotated member 77. Rack teeth (not shown) are provided on the convex surface 78a of the guide arm 78.

The ophthalmic apparatus further includes a main body 79 which is attached to the guide arm 78 to be capable of tilting along the guide arm 78 through a bracket 80 as shown in FIG.1B. A rotational operating knob 81 is provided on the bracket 80. A gear 82 engaging with the rack teeth provided on the convex surface 78a of the guide arm 78 is provided integrally with the rotational operating knob 81. The gear 82 can be driven by a tilting motor 83.

With the above-mentioned structure, the main body 79 is configured to perform forward and backward movement, rightward and leftward movement, horizontally swinging movement, and tilting movement through a drive device including the horizontally rotating motor 76 and the tilting motor 83 and so on. In other words, the main body 79 can be moved in three dimensions.

Meanwhile, a balance mechanism is provided configured to support the main body 79 movably with an upward and downward alight force, although it is not shown. As the balance mechanism, because a well known mechanism may be used, a description thereof is omitted.

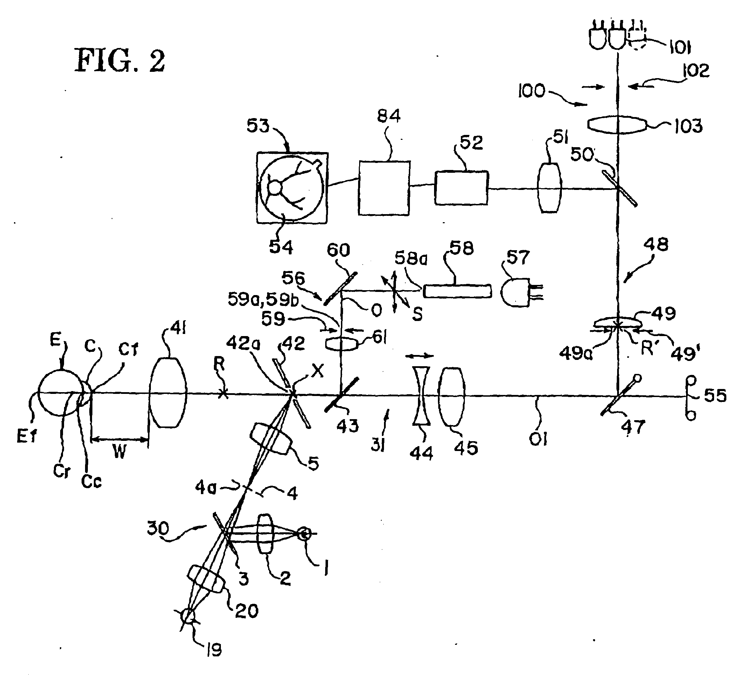

A photographic optical device to photograph a tested eye E of a subject and obtain an image of the tested eye is installed in the main body 79, as shown in FIG.2.

The photographic optical device includes an illumination optical system 30, photographic optical systems 30 and 48, an alignment target optical system 56, and a fixation target photographic optical system 100.

The illumination optical system 30 has an observation light source 1, a condenser lens 2, a dichroic mirror 3, a ring slit plate 4, a relay lens 5, an objective lens 41, and a perforated mirror 42. The dichroic mirror 3 has visible light-permeableness and infrared light-reflectiveness. The ring slit plate 4 has a ring-shaped opening 4a.

Illumination flux emitted from the observation light source 1 is guided to the ring-shaped opening 4a of the ring slit plate 4 through the condenser lens 2 and the dichroic mirror 3, illumination light passed through the ring-shaped opening 4a is once imaged near the perforated mirror 42 through the relay lens 5.

The illumination optical system 30 includes a photographic light source 19 and a condenser lens 20 which are disposed behind the dichroic mirror 8.

When the tested eye is photographed, the photographic light source 19 is emitted. Photographing light emitted from the photographic light source 19 is once imaged near the perforated mirror 42 passing through the condense lens 20 and the dichroic mirror 3, similarly to the illumination light by the observation light source 1.

One photographic optical system 31 includes the objective lens 41, the perforated mirror 42, a half mirror used to reflect the alignment light flux, as mentioned hereinafter, a focusing lens 44, an imaging lens 45, and a flip-up mirror 47. Another photographic optical system 48 includes the ffip-up mirror 47, a dichroic mirror 50 and a television relay lens 51, and constitutes an observation system together with a television camera having a CCD (charge-coupled device) as photographic means, and a television monitor 53.

Light flux reflected on a fundus Ef of the tested eye is guided to the objective lens 41 and imaged on a fundus-conjugate plane conjugating with the fundus Ef through the objective lens 41, thereafter, passes through the opening 42a of the perforated mirror 42 and the half mirror 43, and is guided to the flip-up minor 47 through the focusing lens 44 and the imaging lens 46. The reflection light flux forming an image of the fundus is imaged on a mounting location R' of a field lens 49 by the flip-up mirror 47 again. The re-imaged reflected light flux is received by the television camera 52 through the dichroic mirror 50 and the television relay lens 51, and the fundus image 54 is displayed on a screen of the television monitor 53.

Here, reference number 49a shows an imaging surface of the field lens 49, reference number 49' a field aperture stop disposed adjacently to the imaging surface 49a.

A film 55 is provided at a conjugate position with the field lens 49 with respect to the flip-up mirror 47. In photographing, the flip-up mirror 47 is disposed out of an optical path of the Photographic optical system 31 simultaneously with the emission of the photographic light source 19, thereby the fundus image 54 is imaged and recorded on the film 55.

The alignment target projecting optical system 56 includes an LED (light emitting diode) 57 as an alignment light source, a light guide 58, a reflector 60, a relay lens 61, and the half mirror 43. The LED 57 has a characteristic emitting near infrared light having a central wavelength of 760nm. An exit end or alignment target 58a of the light guide 58 is disposed to position on an optical axis O of the relay lens 61 or optical axis O1 of the photographic optical system 31.

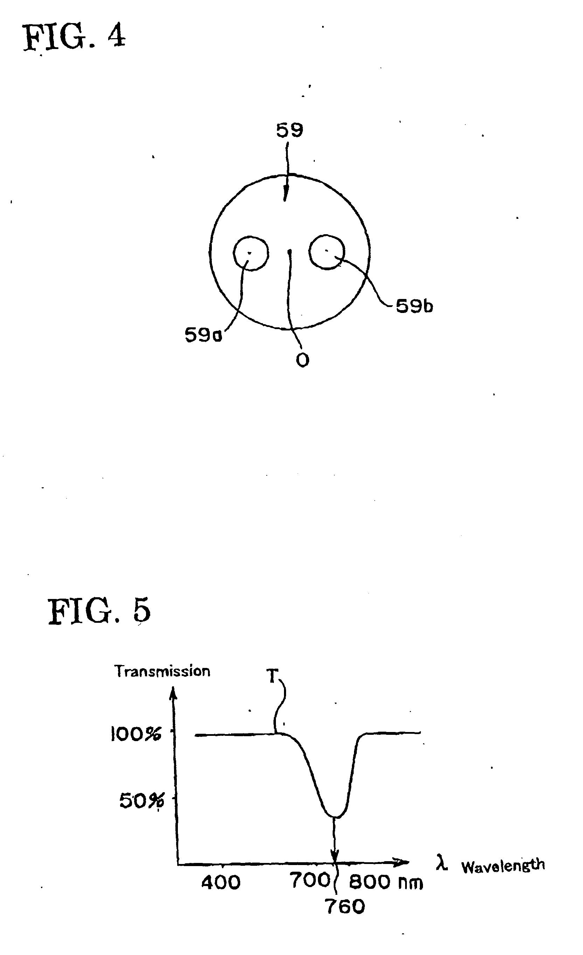

A two opening-aperture stop 59 is disposed between the relay lens 61 and the reflector 60. The two opening-aperture stop 59 includes a pair of openings 59a and 59b, as shown in FIG.4. The openings 59a and 59b are disposed in a symmetry site to an optical axis of the two opening-aperture stop 59. The two opening-aperture stop 59 is disposed close to the relay lens 61.

Alignment light flux emitted from the exit end 58a of the light guide 58 is reflected on the reflector 60 and is guided to the openings 59a and 59b of the two opening-aperture stop 59. Alignment light fluxes passed through the openings 59a and 59b are guided to the relay lens 61. The alignment light fluxes passed through the relay lens 61 are reflected on the half mirror 43 toward the perforated mirror 42.

The relay lens 61 is configured to image the exit end 58a of the light guide 58 on a central position X of the opening 42a of the perforated mirror 42 or optical axis O1 of the photographic optical system 31 once. The half mirror 43 has a translucent characteristic T which passes half of light flux of about wavelength 760mm and passes generally 100% of light flux having wavelengths other than the wavelength. Therefore, an amount of the reflected light flux on the fundus Ef is prevented from lowering under the existence of the half mirror 43.

A pair of alignment light fluxes for forming the alignment target 58a formed on the central position X are guided to the cornea C of the tested eye E through the objective lens 41. Here, when a working distance or operational distance W from the tested eye E to the main body 79 and positions of up, down, right and left directions are adequate, an alignment image is projected and imaged on an intermediate position Cc between the an apex Cf of the cornea C and a curvature center Cr of the cornea C by the pair of alignment light fluxes emitted from the exit end 58a and passed through the openings. On the contrary, when the working distance W from the tested eye to the main body is misaligned with an adequate position, the alignment image based on the pair of alignment light fluxes is divided into two and the divided images are projected separately on the cornea C across the intermediate position Cc.

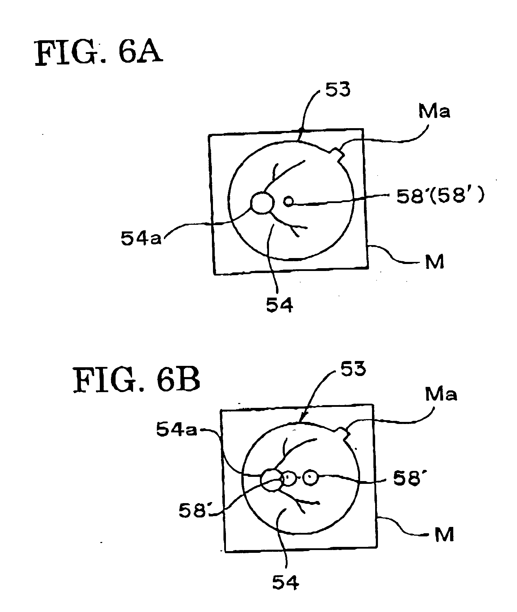

Reflection light flux of the alignment light fluxes reflected on the cornea C is imaged on a fundus conjugate plane R by the objective lens 41 when the working distance W is adequate. The reflection light flux imaged on the fundus conjugate plane R passes the opening 42a and imaged on the television camera 52, similarly to the reflection light flux for forming the fundus image 54. Thereby, an alignment image (image of the exit end 58a) 58' together with the fundus image 54 is displayed on a screen of the television monitor 53, as shown in FIG.6A.

When the working distance W is misaligned with the adequate position, the alignment image or image 58' of the exit end is separated and imaged on the television monitor 53, as shown in FIG.6B. An operator can perform adjustment for aligning the photographic optical system by viewing alignment and separation of the alignment image 58' based on the alignment light fluxes.

A fixation target photographic optical system 100 is provided behind the dichroic mirror 50. The fixation target photographic optical system 100 includes a fixation light source 101 for guiding visual line of the tested eye E, an aperture stop 102 as a fixation target, and a lens 103 for projecting the fixation target. The fixation target is projected on the fundus Ef of the tested eye E through each optical system-element of the photographic optical system 31. The fixation light source 101 comprises a plurality of light sources, for example, five light sources one of which is used for photographing a central portion of the fundus and the others are used for photographing a circumference portion of the fundus. In FIG.2, two fixation light sources 101 for photographing a circumference portion of the fundus are shown, and the remaining two fixation light sources 101 for photographing a circumference portion of the fundus are disposed in a direction perpendicular to paper. Accordingly, the remaining two fixation light sources 101 are not shown in the drawings.

When the central portion of the fundus is photographed, one of the fixation light sources 101 used for photographing the central portion of the fundus is selected by a fixation target selection switch (not shown) and lighted to present the fixation target on the tested eye E. When photographing a circumference portion as in each of up, down, right and left portions and so on of the fundus, the fixation light source 101 corresponding to the circumference portion of the fundus desired to photograph is lighted when photographing the circumference portion to present the fixation target on the tested eye E.

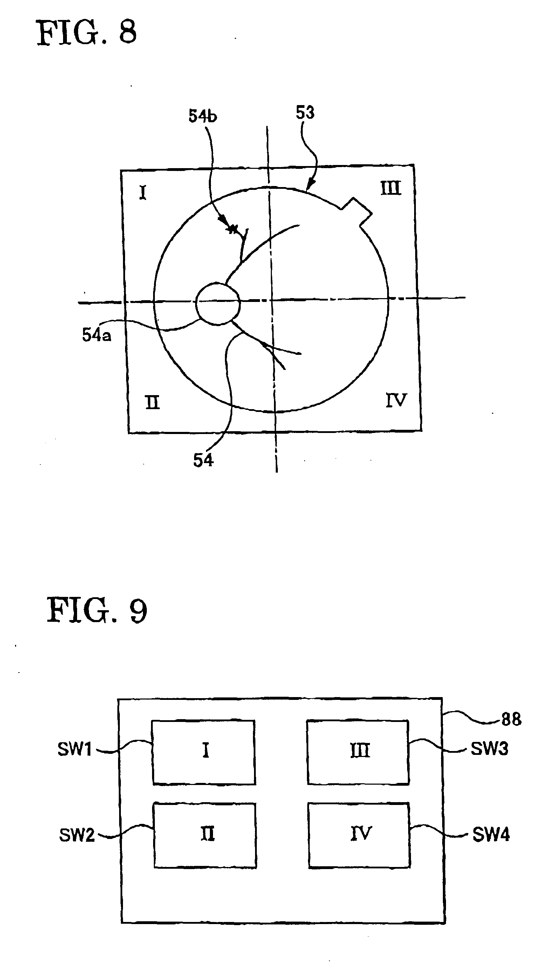

In addition, a display, I scale I to determine a degree of opening of pupil of tho tested eye is synthetically displayed on a central portion of the screen of the television monitor 53 (see FIG.8). The I scale I is also used as a reference position mark of the alignment image when photographing the central portion of the fundus.

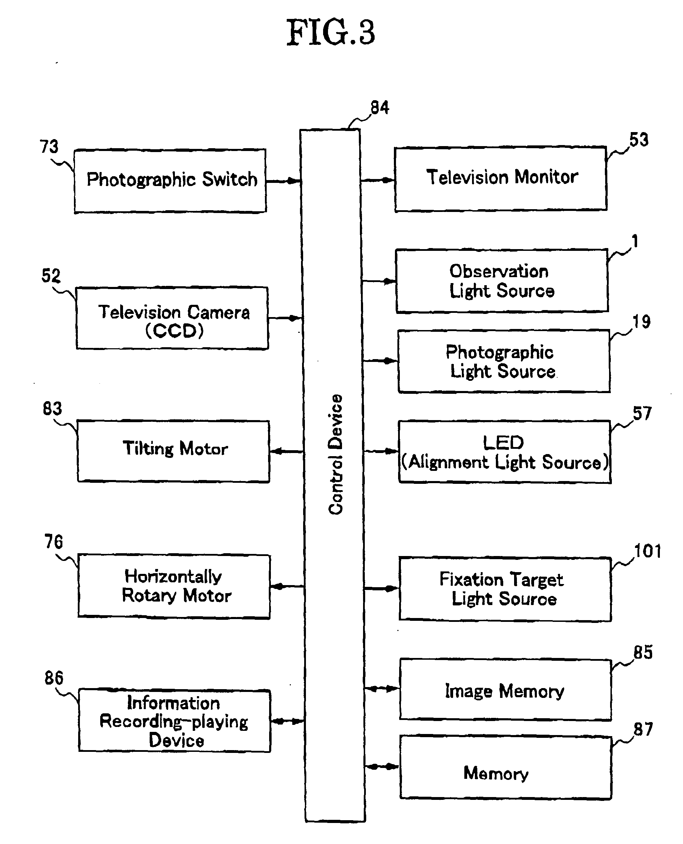

The observation light source 1, the photographic light source 2, the alignment light source (LED) 57 and the fixation light source 101 as shown in FIG.2 are configured so that lighting is controlled by a control device 84 shown in FIG.3. An image signal (picture signal) based on an image photographed by the television camera 52 and an ON signal from the photographic switch 73 are input in the control device 84.

The control device 84 controls the flip-up mirror 47 to be raised by a drive device (not shown) so that the flip-up mirror is out of the optical path and the fundus image is imaged on the film 55, when the photographic switch 73 is pressed and the ON signal is input in the control device 84 in a photographic mode. In addition, the control device 84, when the photographic switch 73 is pressed and the ON signal is input in the control device 84 in an electro-photographic mode, is configured to photograph the fundus image by controlling the television camera 52 and to acquire an electro-still image of the fundus. Switching of the photographic mode and the electro-photographic mode is carried out by a mode-switching mechanism (not shown).

In this case, the control device 84, when photographing the electro-still image of the fundus, is configured to display the electro-still image on the television monitor 53 and store the electro-still image in an image memory 85.

Moreover, the control device 84 is configured to record the still image of the fundus in an information recording-playing device 86 such as a hard disc, magnetic optical disc or the like. Furthermore, the control device 84 is configured to be capable of recording control data, control information or the like of each part of the photographic optical device in a memory 87 such as a RAM or the like.

Furthermore, the control device 84 is configured to display the observation image photographed by the television camera 52 on the television monitor 53 with real time. Because a well known structure can be used for such a structure, a detailed description thereof is omitted.

In addition, the control device 84 is configured to receive an operating signal from a horizontally rotating operation switch (not shown) and control the horizontally rotary motor 76 to perform normal or reverse rotation and receive an operating signal from a tilting switch (not shown) and control the tilting motor 83 to perform normal or reverse rotation.

Meanwhile, the control device 84 controls a drive device (not shown) such as a pulse motor or the like to perform normal or reverse rotation by rotating operation of the joystick 72 about the axis, thereby the arm 74 is driven to move up and down

The drive device is configured to move at least the photographic optical systems 31 and 48 relative to the tested eye E so that their optical axes align with each other. The movement is controlled by the control device as follows. In illustrated embodiment, the drive device is configured to drive the main body 79 containing the photographic optical systems 31 and 48, the alignment target projecting optical system 56 and so on. Because a well known structure can be used for the drive device, a detailed description is omitted.

Hereinafter, operation of the above-mentioned ophthalmic apparatus is described about a case of photographing the central portion of the fundus and a case of photographing the circumference portion of the fundus.

### (1) Case of photographing the central portion

When photographing the central portion of the fundus, one of the fixation light sources 101 to photograph the central portion of the fundus is selected by the fixation target selection switch (not shown) and lighted. Thereby, visual line of the subject is aligned with the optical axis O1 of the photographic optical system 31.

On the other hand, as mentioned above, the alignment light flux emitted from the exit end 58a of the light guide 58 is reflected on the reflector 60 and guided to the openings 59a and 59b of the two opening-aperture stop 59. The light fluxes passed through the openings 59a and 59b are guided to the relay lens 61. The alignment light fluxes passed through the relay lens 61 are reflected on the half mirror 43 toward the perforated mirror 42.

The relay lens 61 is configured to image the exit end 58a of the light guide 58 on the central position X of the opening 42a of the perforated mirror 42 or optical axis O1 of the photographic optical system 31 once. The pair of alignment light fluxes for forming the alignment target 58a and formed on the central position X of the opening 42a of the perforated mirror 42 are guided to the cornea C of the tested eye E through the objective lens 41. The alignment reflection light flux reflected on the cornea C is imaged on the television camera 52, similarly to the reflection light flux for forming the fundus image 54, thereby the alignment image 58' (image of exit end 58a) together with the fundus image 54 is displayed on the screen of the television monitor 53, as shown in FIGs.6A and 6B.

Here, when the working distance W from the tested eye E to the main body 79 is misaligned with the adequate position, the alignment image based on the pair of alignment light fluxes is divided into two and divided images are projected separately on the cornea C across the intermediate position Cc.

In this way, when the working distance W is misaligned with the adequate potion, the alignment image 58' or image of the exit end 58a is in a state separated into two, thereby the alignment image is displayed on the television monitor 53 in an out-of-focus state.

In this state, because the alignment light flux enters the cornea C of the tested eye E obliquely, the reflection light flux on the cornea C results in flare.

Consequently, the operator moves the main body 79 forwardly and backwardly by the joystick 72 so that the alignment image 58' is in a focused state, as shown in FIG.6A. In addition, the operator allows the alignment image 58' to align with the I scale I displayed on the television monitor 53 by moving the main body 79 in up, down, right and left directions.

When the working distance W from the tested eye E to the main body 79, the positions of up, down, right and left directions of the main body are adequate and the optical axis O1 of the photographic optical system 31 aligns with the optical axis of the tested eye, the alignment image is imaged on the intermediate position Cc between the apex Cf of the cornea C and the curvature center Cr of the cornea C by the pair of alignment light fluxes emitted from the exit end 58a and passed through the openings.

In this case, the reflection light flux of the alignment light flux reflected on the cornea C is imaged on the fundus conjugate plane R by the objective lens 41 when the working distance W is adequate. The reflection light flux imaged on the fundus conjugate plane R passes through the opening 42a and imaged on the television camera 52, similarly to the reflection light flux forming the fundus image 54. Thereby, the alignment image 58' (image of the exit end 58a) is displayed on the screen of the television monitor 53 at one with the fundus image 54.

In this state, because the alignment light flux enters the cornea C of the tested eye E from front, flare does not occur in the reflection light flux on the cornea C.

Accordingly, the control device 84 determines that alignment to focus the alignment image 58' is completed when the state in which the alignment image 58' is focused on the screen of the television monitor 53 as one image is detected by an output signal from the CCD (not shown) of the television camera 52, and the control device is adapted to light the photographic light source 19 and photographs the fundus image 54. The control device 84 then stores the photographed fundus image 54 in the image memory 85, displays it on the screen of the television monitor 53, and stores it in the information recording-playing device 86.

### (2) Case of generating flare by alignment flux

In this way, when the central portion of the fundus is photographed, if the tested eye is normal, the working distance W from the tested eye E to the main body 79 is adequate, and the optical axis O1 of the photographic optical system 31 aligns with the optical axis of the tested eye E, the flare in the reflection flux of the alignment flux on the cornea C does not occur, therefore there are no affections of the flare on the photographed fundus image 54.

### (a) Case where the tested eye is not normal for heterophoria or the like

### (a1) Control of the optical axis O1 of the photographic optical system 31 to align with the optical axis of the tested eye E

However, even if the subject faces front and the main body 79, when the tested eye E faces substantially obliquely for the heterophoria or the like, the optical axis O1 of the photographic optical system 31 does not align with the optical axis of the tested eye E although the working distance W from the tested eye E to the main body 79 is adequate, the alignment light flux enters the cornea C of the tested eye E obliquely. In this case, the flare occurs in the reflected light flux of the alignment light flux on the cornea C. The flare affects the fundus image 54.

Meanwhile, when the central portion of the fundus Ef is photographed, a papillary portion 54 a of the fundus image 64 having no flare is bright, whereas portions other than the papillary portion are dark. It can be identified which of the right and left eyes has the papillary portion 54a. The photographed fundus image 54 can be identified by a cutout mark of a mask (not shown) used in photographing the tested eye E. In other words, as shown in FIGs.6A and 6B, a mask image M together with the fundus image 54 is photographed and a cutout mark image Ma is provided on the mask image M. The cutout mark image Ma is right and left reveisal in the right and left fundus images.

On the other hand, if there is flare in the fundus image, a portion of flare is bright. If portions other than the papillary portion 54a are brighter than the fundus image 54 having no flare, it can be determined that the flare occurs in the other portions. The determination can be achieved based on the fundus image 54 which is a moving image when observing the photograph by the television camera 52 through the control device 84.

In this case, the control device 84 horizontally rotates the main body 79 about the supporting shaft 75 rightward and leftward by controlling the horizontally rotary motor 76 to perform the normal or reverse rotation in a direction where the optical axis O1 of the photographic optical system 31 aligns with the optical axis of the tested eye E, and moves the main body 79 upwardly and downwardly along the circular-are guide arm 78 by controlling the tilting motor 83 to perform the normal or reverse rotation, based on the variation of contrast of all the moving image which is the fundus image 54 photographed by the television camera 52, if the flare occurs in the fundus image 54.

The control device 84 is configured to light the photographic light source 19 and photograph the fundus image 54, when the flare in the fundus image 54 is equal to or lesser than a predetermined threshold. The control device 84 is configured to store the photographed fundus image 54 in the image memory 85, display it on the screen of the television monitor 53, and record it in the information recording-playing device 86.

### (a2) Determination of contrast variation by control of the main body 79

Moreover, as a method other than the above, the control device 84 may store the contrast variation of the moving image which is the fundus image 54 and the moving position of the main body 79 in the image memory 87 as needed, while horizontally rotating the main body 79 about the supporting shaft 75 rightward and leftward by controlling the horizontally rotary motor 76 to perform the normal or reverse rotation, and moving the main body 79 upwardly and downwardly along the circular-arc guide arm 78 by controlling the tilting motor 83 to perform the normal or reverse rotation, based on the variation of contrast of all the moving image which is the fundus image 54 photographed by the television camera 52, if the flare occurs in the fundus image 54.

In this case, the control device 84 is configured to obtain a position of the main body 79 where the flare is minimum based on the moving position of the main body 79 and the contrast variation of the moving image of the fundus image 54 which are stored in the image memory 87, move the main body 79 at the obtained position, light the photographic light source 19, and photograph the fundus image 54. In addition, the control device 84 stores the photographed fundus image 54 in the image memory 85, displays it on the screen of the television monitor 53, and stores it in the information recording-playing device 86.

### (b) Case where the cornea of the tested eye E strains

If the cornea strains because of disease of cornea and so on, flare occurs when the alignment light flux is reflected on the cornea. The flare affects the fundus image 54. In this case, the movement of the main body 79 is controlled as mentioned in the above (a2) to photograph and record the fundus image 54.

### (c) Case of a subject having a tested eye of pupil lesser than the standard

Furthermore, when the subject has the tested eye E which has pupil lesser than the standard, even if the central portion of the fundus Ef is photographed, the alignment light flux is cut by iris of the tested eye E, therefore affection of the flare tends to generate on a circumference portion of the fundus image 54. Even in this case, if the fundus image 74 on the television monitor 53 is previously divided into first to fourth quadrants, as shown in FIG.8, for example, when there is a diseases part 54b in the first quadrant, if it is possible to photograph so that flare does not in at least the part, the determination of the diseases is possible.

For example, by providing an operational panel 88 having operational switches sw1, sw2, ew3 and sw4 corresponding to the four quadrants, respectively, as shown in FIG.9 on the movable base 71 as shown in FIGs.1A and 1B, the main body 79 may be moved by controlling the motors 76 and 83 by means of the control device 84 so that flare occurs in a quadrant corresponding to a pressed operational switch of the operational switches, but flare does not occur in a quadrant which is symmetric with respect to a point to the quadrant corresponding to the pressed operational switch of the operational switches.

On the contrary, the main body 79 may be moved by controlling the motors 76 and 83 by means of the control device 84 so that flare does not occur in a quadrant corresponding to the pressed operational switch of the operational switches, but flare occurs in a quadrant which is opposite (side symmetric with respect to the point) to the quadrant corresponding to the pressed operational switch of the operational switches. In other words, if there is the disease part 54b in the quadrant, as mentioned above, the main body 79 may be moved by controlling the motors 76 and 83 by means of the control device 84 so that flare occurs in the quadrant by pressing the switch sw1, but flare does not occur in the quadrant having the disease part 54b.

Instead of providing the operational switches sw1, sw2, sw3 and sw4, the screen of the television monitor 59 is formed into a touch panel to which a finger of the operator can touch, thereby, it can be structured that the flare does not occur in a touched portion or opposite potion thereto so that the disease part has no flare.

### (3) Case of forming a panorama fundus image by photograph of a circumference portion of fundus

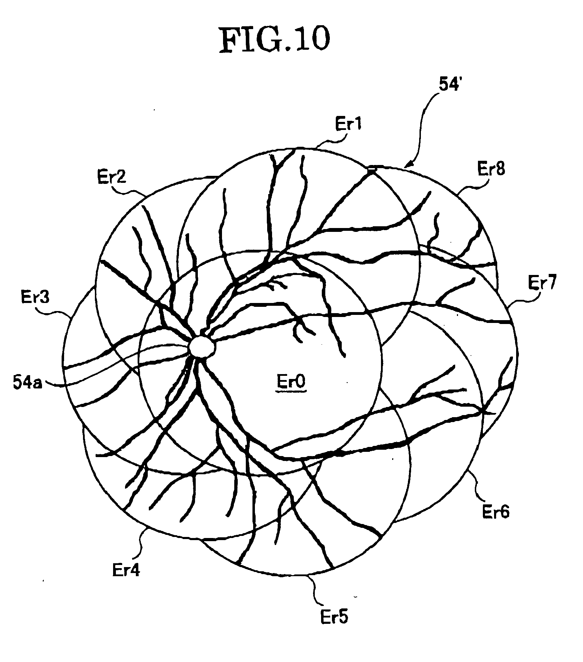

In this case, an example in which an upper side of the fundus Ef of the tested eye E, that is to say, an image Er0 that the papillary part 54a of the tested eye is disposed on a left position of a screen is placed on a central position, and eight circumferential images Er1 to Er8 of the fundus about the image Er0 are imaged to form the panorama fundus image 54', as shown in FIG.10 is first described below.

In this case, as mentioned in the above (1), the fundus image 54 of the central portion of the fundus Ef is first imaged to obtain the image Er0. Basically, flare does not occur in the image Er0.

On the other hand, the circumferential images Er1 to Er8 of the fundus are in a state generating the flare, because the alignment light flux enters the cornea C obliquely in order to photograph the tested eye while moving the visual line of the subject in up, down, oblique up and down, right and left directions and so on by use of the fixation target light source 101.

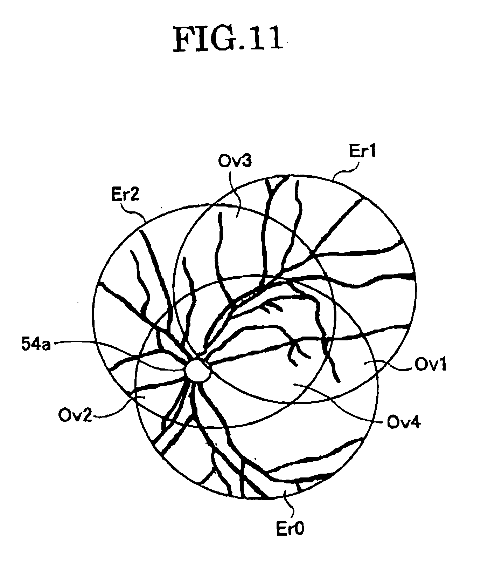

Here, an example in which the images Er1 to Er8 are imaged on the image Er0 in sequence is described by use of, for example, three images Er0 to Er3, as shown in FIG. 11.

Assuming that an overlapped portion of the images Er0 and Er1 only is Ov1, an overlapped portion of the images Er0 and Er2 only Ov2, an overlapped portion of the images Er1 and Er2 only Ov3, and an overlapped portion of the images Er0 to Er3 Ov4, because the central image Er0 may be used for an image of the overlapped portion Ov4, even if flare occurs in the overlapped portion Ov4 of the images Er0, Er1 and Er2, there are no any problems.

Accordingly, when the images Er1 and Er2 are photographed, the main body 79 is adapted to be moved by the motors 76 and 83 which are controlled by the control device 84 so that it is permitted that the flare occurs in the overlapped portion Ov4.

Next, some modifications of the ophthalmic apparatus according to the Present invention are described.

### (Modification 1)

In the above-mentioned ophthalmic apparatus, only the arm 74 is moved upwardly and downwardly by the drive motor (not shown) interconnecting with the rotational operation (normal or reverse rotation) about the axis of the joystick 72. The ophthalmic apparatus is not necessarily limited to the structure.

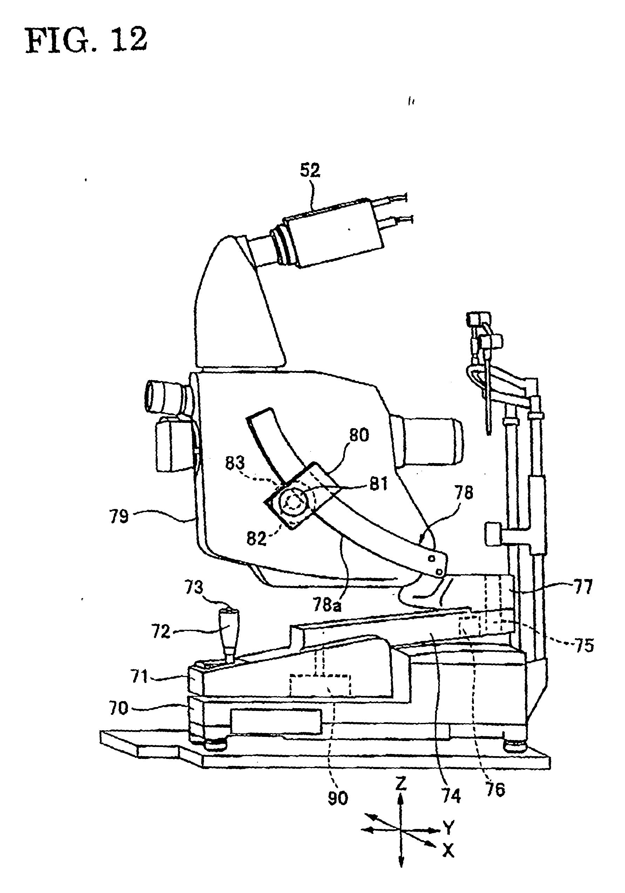

For example, by providing an alignment drive mechanism 90 to drive the main body 79 in X, Y and Z directions (X shows right and left directions, Y shows back and front directions, Z shows up and down directions), as shown in FIG.12, the main body 79 can be moved by the alignment drive mechanism 90 in the X, Y and Z directions.

In this case, the alignment drive mechanism 90 includes an X direction drive mechanism (not shown) to drive the movable base 71 in X direction, a Y direction drive mechanism (not shown) to drive the movable base 71 in Y direction, and a Z direction drive mechanism (not shown) to drive the arm 71 in Z direction relative to the movable base 71.

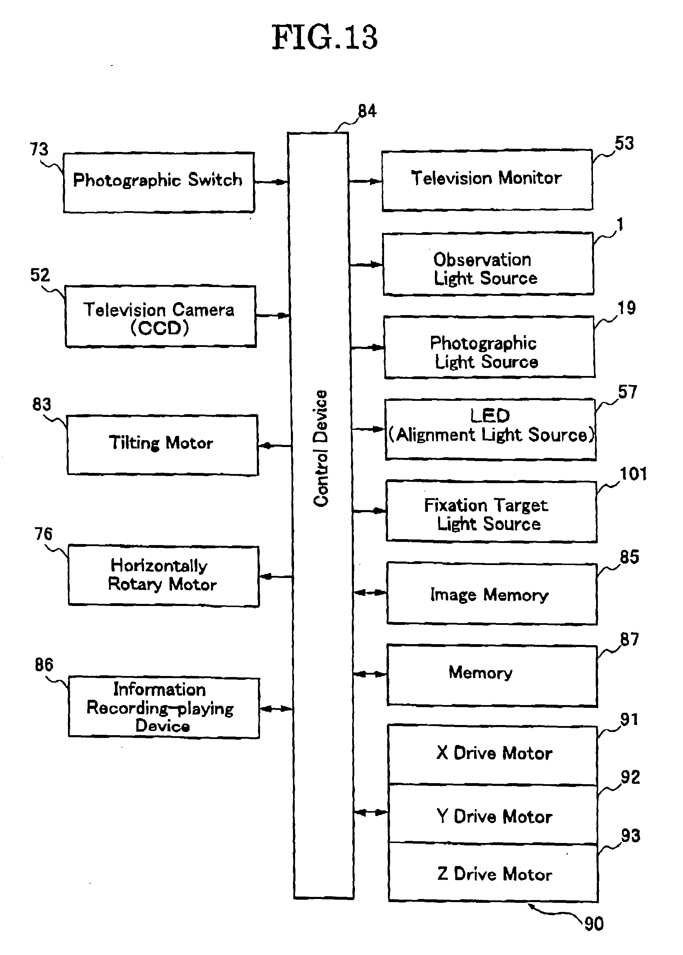

As shown in FIG.13, the X direction drive mechanism has an X drive motor 91 such as a pulse motor or the like, the Y direction drive mechanism has a Y drive motor 92 such as a pulse motor or the like, and the Z direction drive mechanism has a Z drive motor 93 such as a pulse motor or the like. The X drive motor 91 is adapted to perform normal or reverse rotation by operation of the joystick 72 in right and left directions to move the movable base 71 in the right and left directions through a rack and gear mechanism (not shown). The Y drive motor 92 is adapted to perform normal or reverse rotation by operation of the joystick 72 in back and front directions to move the movable base 71 in the back and front directions through a rack and gear mechanism (not shown). The Z drive motor 93 is adapted to perform normal or reverse rotation by rotational operation of the joystick 72 about the axis to move the arm 74 in the up and down directions through a feeding screw mechanism (not shown). Because a well known three-dimensional drive mechanism can be used for the alignment drive mechanism 90, a detailed description thereof is omitted.

The motors 91 to 93 of the alignment drive mechanism 90 are configured to be controlled by the control device 84, as shown in FIG.13.

In addition, because the control device 84 drives the main body 79 in a direction reducing the flare, the control device is adapted to control the motors 91 to 93 of the alignment drive mechanism 90 so that the flare in portions other than the overlapped portions of the images Er1 to Er8 on the image Er0 decreases, based on a bright point image photographed by the television camera 52, without controlling the drive of the tilting motor 83 and the horizontally rotary motor 76. In other words, the control device 84 controls the motors 91 to 93 of the alignment drive mechanism 90 to generate the flare in the overlapped portions of the images Er0 and Er1 to Er8 and to reduce the flare in the portions other than the overlapped portions.

Even in the modification 1, by controlling the motors 91 to 93 by the control device 84 and driving the main body 79 in the X, Y and Z directions only, the flare decreases, similarly to the case of driving the above-mentioned tilting motor 83 and the horizontally rotary motor 76.

### (Modification 2)

In addition to the modification 1, the main body 79 can be moved in a direction of further reducing the generation of flare by controlling the above-mentioned tilting motor 83 and the horizontally rotary motor 76, similarly to the above.

### (Modification 3)

Furthermore, in the above-mentioned modifications, when the circumference images Er1 to Er8 are photographed to form the panorama fundus image, the tilting motor 83 and the horizontally rotary motor 76 are controlled to generate the flare in the overlapped portions of the images Er0 and Er1 to Er8, but the ophthalmic apparatus is not necessarily limited to the structure.

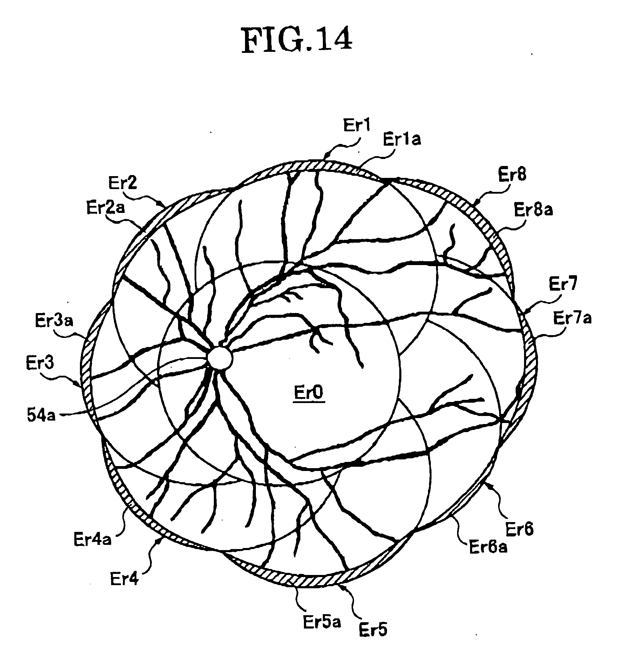

For example, as shown in FIG.14, the main body may be moved to generate flare in circumference portions opposite to the overlapped portions of the images Er0 and Er1 to Er8 or portions Er1a to Er8a as shown in diagonal lines. In this case, basically, the control device controls only the drive of the motors 91 to 93 and controls the main body 79 in three dimensions (X, Y, and Z directions). Thereby, it is possible to generate the flare in the portions Er1a to Er8a which are the circumference portions of the images Er1 to Er8, shown by the diagonal lines.

Also, in addition to the modification 2, by controlling the tilting motor 83 and the horizontally rotary motor 76, it is possible to generate the flare in the circumference portions Er1a to Er8a of the images Er1 to er8.

In this way, a designation device is structured to allow the control device to designate a position where the flare occurs so that the photographic optical system is driven and controlled by the control device in which the generated position of the flare is designated.

The designation device may be installed in the control device 84. Alternatively, the designation device can be structured by combination of a plurality of switches (not shown) to designate the photographed positions for the panorama fundus images and the control device 84.

By the above-mentioned modifications, even if the flare occurs in the fundus image, it is possible to accomplish the alignment of the photographic optical system which does not affect diagnosis of the diseases part, similarly to the above-mentioned embodiments.

Although the preferred embodiments and the modificatione of the present invention have been mentioned, the present invention is not limited to the embodiments and the modifications, it should be noted that further various modifications and changes can be made to these embodiments and the modifications.

For example, although the present invention has been applied to the photograph of the fundus of the tested eye, it is possible to apply the present invention to a case of measuring a refracting power of the tested eye or photographing corneal endothelial rolls.

## Claims

1. An ophthalmic apparatus, comprising:
a photographic optical system (31, 48) capable of photographing a tested eye (E) and obtaining an image of the tested eye:
a drive device (76, 83, 90) configured to drive the photographic optical system relative to the tested eye in three dimensions;
an alignment target projecting optical system (56) to project alignment light flux to the tested eye; and
a control device (84) configured to control the drive device based on a state of reflection light flux formed by reflection of the alignment light flux projected by the alignment target projecting optical system on the tested eye,
**characterized in that**
the control device (84) is configured, when flare occurs in the reflection light flux of the alignment light flux reflected on the tested eye, to control the drive device to move the photographic optical system in a direction where the flare decreases.

2. The ophthalmic apparatus according to claim 1,
**characterised in that** at least the photographic optical system is contained in a movable main body (79).

3. The ophthalmic apparatus according to claim 1,
**characterized in that** the alignment light flux is projected on cornea (C) of the tested eye.

4. The ophthalmic apparatus according to claim 1,
**characterized in that** the alignment target projecting optical system (56) is configured to form a bright point image of the reflection light flux of the alignment light flux reflected on the tested eye,
**characterized in that** the control device (84) is configured to perform alignment of the tested eye and the photographic optical system based on the bright point image of the reflection light flux.

5. The ophthalmic apparatus according to claim 1.
**characterized in that** the photographic optical system (31, 48) includes an observation and photograph camera to observe and photograph the tested eye.

6. The ophthalmic apparatus according to claim 6,
**characterized in that** the control device (84) is configured to detect the flare by the alignment light flux through the observation and photograph camera, and control the drive device to move the photographic optical system in the direction where the flare decreases.

7. The ophthalmic apparatus according to claim 1,
further comprising a designation device configured to allow the control device to designate a position where the flare occurs, when the flare by the alignment light flux occurs and control the drive device so that the photographic optical system is driven and controlled by the control device in which the generated position of the flare is designated.

8. The ophthalmic apparatus according to claim 1,
**characterized in that** the drive device (76, 83, 90) includes a horizontally rotary mechanism (76, 77) to rotate the photographic optical system horizontally and a tilting drive mechanism (82, 83) to move the photographic optical system, wherein the control device is configured to control the horizontally rotary mechanism and the tilting drive mechanism in the direction where the flare by the alignment light flux decreases.
